# EUROPÄISCHE PATENTSCHRIFT

(11) **EP 0 884 031 B1**
(45) Veröffentlichungstag und Bekanntmachung des Hinweises auf die Patenterteilung: **15.01.2003**
(21) Anmeldenummer: 98109681.1
(22) Anmeldetag: 27.05.1998
(51) Int. Cl.: A61F 2/16

(54) **Kapseläquatorring**
Equatorial ring for capsular bag
Anneau équatorial pour capsule cristallinienne

(30) Priorität: 09.06.1997 DE 19724108
(43) Veröffentlichungstag der Anmeldung: 16.12.1998
(73) Patentinhaber: Morcher GmbH, D-70374 Stuttgart (DE)
(72) Erfinder: Nishi, Okihiro M.D., Katano-shi, Osaka 576 (JP)
(74) Vertreter: Patentanwalts-Partnerschaft, Rotermund + Pfusch + Bernhard

(56) Entgegenhaltungen:
- EP-A- 0 507 292
- FR-A- 2 754 173
- US-A- 5 628 795

## Beschreibung

Die Erfindung betrifft einen Kapseläquatorring, welcher in den eröffneten Kapselsack eines Auges implantierbar ist und im implantierten Zustand mit seinem Außenumfang an der Innenseite des Kapselsackes an dessen Äquator anliegt und den Kapselsack radial stabilisiert, mit den Merkmalen des Oberbegriffs des Anspruches 1.

Falls die natürliche Linse eines Auges, beispielsweise aufgrund einer starken Trübung, operativ entfernt werden muß, soll der die natürliche Linse aufnehmende Kapselsack, welcher zur Entfernung der natürlichen Linse auf der der Iris zugewandten Vorderseite eröffnet wird, erhalten bleiben, um die anatomischen Verhältnisse im Auge möglichst wenig zu verändern und die Implantation einer künstliche intraocularen Linse zu erleichtern.

Allerdings muß bei der Entfernung der natürlichen Linse mit mehr oder weniger umfangreichen Beschädigungen von Muskel- bzw. Fasersträngen gerechnet werden, welche den Kapselsack im Bereich seines Äquators außenseitig innerhalb des Auges haltern. Um die damit verbundenen Verformungen des Kapselsackes bzw. übermäßige Beanspruchungen der unbeschädigt gebliebenen Muskel- bzw. Gewebefasern zu vermeiden, ist es bekannt, im eröffneten Kapselsack einen Kapseläquatorring der eingangs angegebenen Art zu implantieren.

Derartige Stabilisierungselemente sind beispielsweise aus der EP 0 507 292 A1 bekannt. Gemäß einer ersten Ausführungsform kann dabei der Kapseläquatorring als geschlossener, faltbarer Ring ausgebildet sein, so daß sich der Ring trotz seiner geschlossenen Form durch relativ kleine Operationsöffnungen am Auge hindurch in den Kapselsack einsetzen läßt. Für einen guten Sitz sollte die Größe des Kapseläquatorringes jedoch möglichst genau an den Kapselsack angepaßt sein.

Gemäß einer zweiten Ausführungsform kann der Kapseläquatorring gemäß der EP 0 507 292 A1 auch als offenes Ringteil mit ineinander steckbaren Enden ausgebildet sein, um eine Anpassung an den Äquatorumfang des jeweiligen Kapselsackes zu ermöglichen.

Der Querschnitt der Ringe der EP 0 507 292 A1 kann scharfkantig quadratisch bzw. mit scharfen Kanten zwischen dem Außenumfang und den Stirnseiten oder mit abgerundeten Kanten bzw. kreisbogenförmig ausgebildet sein.

Aus der EP 0 478 929 A1 ist es bekannt, an den miteinander zu verbindenden Enden eines offenes Kapseläquatorringes gegengleiche Sägezahnprofile auszubilden, um eine besonders belastbare Verbindung zu ermöglichen.

Gemäß der US 5 628 795 sollen die Kapselsackringe vorzugsweise eine toroidförmige Außenseite aufweisen, wobei gegebenenfalls auch eine C-förmig offene Ringform vorgesehen sein kann. Gemäß der US 5 628 795 ist jedoch auch ein rechteckiger Querschnitt möglich, wobei die langen Rechteckseiten von den Stirnseiten des Ringes gebildet werden.

Aufgabe der Erfindung ist es, einen Kapseläquatorring zu schaffen, bei dem die Wahrscheinlichkeit von postoperativen Migrationen der Linsenepithelzellen am Kapselsack besonders niedrig und die Implantation der künstlichen Linse leicht möglich ist.

Diese Aufgabe wird mit einem Kapseläquatorring der eingangs angegebenen Art gelöst, wenn dieser die kennzeichnenden Merkmale des Anspruches 1 aufweist.

Die Erfindung beruht auf dem allgemeinen Gedanken, einen Kapseläquatorring zu schaffen, welcher sich einerseits aufgrund seiner Federbügelcharakteristik der Größe des jeweiligen Kapselsackes ohne weiteres anzupassen vermag und sich andererseits mit merklicher Spannung an den Äquator des Kapselsackes anlegt. In Verbindung mit dem Profil des Ringes, welches beidseitig der Äquatorebene des Kapselsackes eine relativ scharfe Ringkante bildet, werden dann im Bereich dieser Kanten linienförmige Zonen des Kapselsackes einer besonders hohen Flächenpressung ausgesetzt, welche noch erhöht werden kann, wenn die Breite des Kapseläquatorringes in Richtung der Ringachse gemäß einer bevorzugten Ausführungsform der Erfindung groß bemessen ist.

Warum damit ein signifikanter inhibitorischer Effekt auf die Migration der Linsenepithelzellen ausübt wird, ist bislang noch nicht endgültig geklärt. Jedoch ist die durch den erfindungsgemäßen Kapseläquatorring bewirkte Knickung des Kapselsackes an den Ringkanten wesentlich. Diese Knickung führt zu einer Diskontiunität am Kapselsack, die eine Proliferation von Linsenepithelzellen auf dem vom Kapselsack gebildeten Leit- bzw. Gerüstwerk verhindert. Jedenfalls hat sich bei In-vitro-Kulturen von Linsenepithelzellen gezeigt, daß eine Migration dieser Zellen an einer Gefäßwand an winkelförmigen bzw. geknickten Übergängen zu einer anschließenden Gefäßwand aufgehalten wird.

Die axiale Breite des Kapseläquatorringes am Äquator des Kapselsackes ist unter anderem deshalb vorteilhaft, weil der Kapseläquatorring wirksam in eine Parallellage zur Äquatorebene gedrängt wird und damit zu einer gleichförmigen Spannung am Äquatorbereich des Kapselsackes führt, ähnlich wie es bei vorhandener natürlicher Linse der Fall ist. Darüber hinaus wird eine innenseitige Berührung zwischen Vorderwand- und Hinterwandteilen des Kapselsackes am Äquatorbereich verhindert, so daß keine Verwachsungen auftreten können. Im übrigen bietet die Erfindung den Vorteil, daß eine zu implantierende künstliche intraoculare Linse, insbesondere eine "faltbare" Linse aus weichem Material, leichter in optimaler Lage implantierbar ist bzw. aufgrund ihrer Elastizität selbsttätig in die optimale Lage "rutscht".

An den freien Enden dieses Federbügels bzw. am Kapseläquatorring sind auf dessen Innenseite kleine Ösen angeformt, um das Element bzw. den Ring bei der Implantation leichter ergreifen und manipulieren zu können.

In Achsansicht des Federbügels besitzen die an die freien Enden anschließenden Teilstücke des Federbügels gegenüber einem mittleren, etwa halbkreisförmigen Teilstück eine verminderte Dicke.

Vorzugsweise verjüngen sich die vorgenannten Teilstücke zu den freien Enden hin.

Im übrigen wird hinsichtlich bevorzugter Merkmale der Erfindung auf die Ansprüche sowie nachfolgende Erläuterung eines besonders bevorzugten Ausführungsform verwiesen, die anhand der Zeichnung beschrieben wird.

Dabei zeigt
- Fig. 1: eine Draufsicht (Achsansicht) eines erfindungsgemäßen Kapseläquatorringes,
- Fig. 2: eine Seitenansicht und
- Fig. 3: einen schematisierten Längsschnitt eines Auges mit im eröffneten Kapselsack implantierten Kapselspannring.

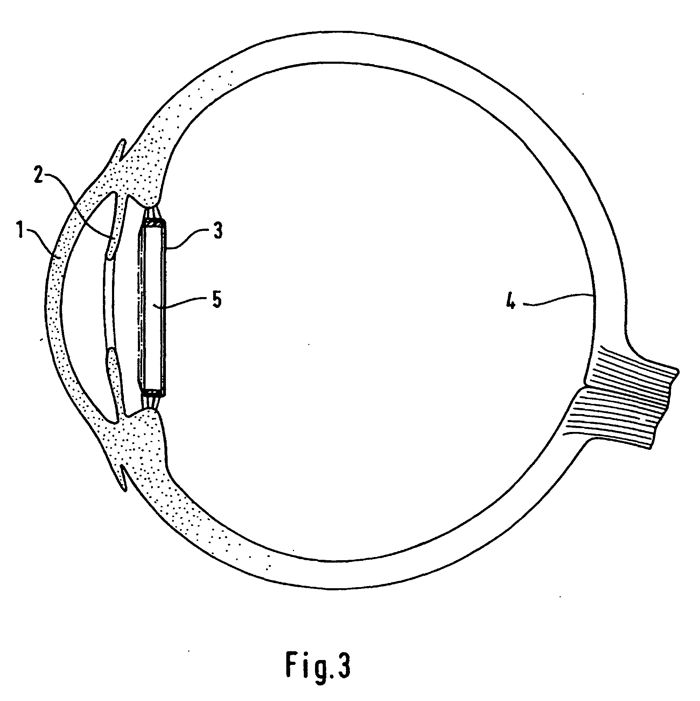

Gemäß Fig. 3 besitzt das dort dargestellte Auge in bekannter Weise eine Hornhaut 1, eine Iris 2, einen normalerweise die natürliche Linse aufnehmenden Kapselsack 3 sowie eine Retina 4.

Im dargestellten Beispiel ist die natürliche Linse entfernt. Dazu ist der Kapselsack 3 auf seiner der Iris 2 zugewandten Seite eröffnet. Bei dieser Operation können Muskel- bzw. Gewebefasern, welche den Kapselsack 3 an seinem Äquator innerhalb des Auges haltern, mehr oder weniger stark beschädigt werden.

Um damit einhergehende Verformungen des Kapselsackes 3 sowie Überlastungen der unbeschädigten Muskel- bzw. Gewebefasern zu vermeiden, kann in den Kapselsack 3 ein Kapseläquatorring 5 eingesetzt werden.

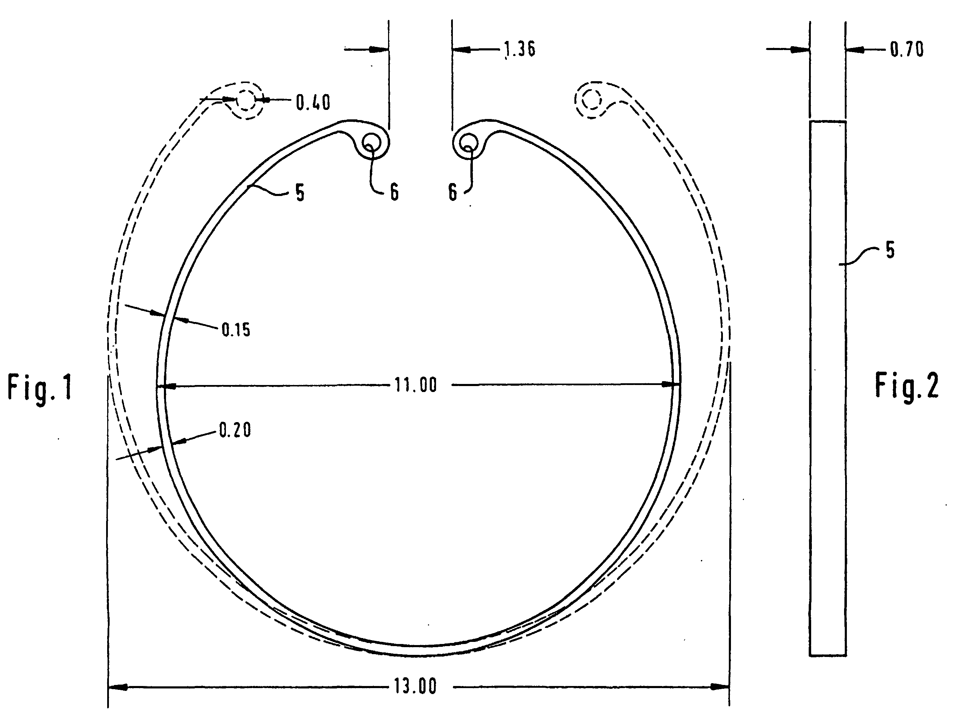

Gemäß den Fig. 1 und 2 ist der Kapseläquatorring 5 als C-förmig offenes Ringteil ausgebildet, welches aus einem elastisch federnden Kunststoff, beispielsweise PMMA (Polymethylmethacrylat) bzw. Acrylglas oder einem sonstigen gut verträglichen Material, wie z.B. Polycarbonat, besteht, wobei optische Materialeigenschaften nebensächlich sind.

Dabei besitzt der Kapseläquatorring 5 einen im wesentlichen rechteckigen Querschnitt, wobei die langen Rechteckseiten parallel zur Ringachse erstreckt sind und zwischen dem Außenumfang und den Stirnseiten des Ringes 5 ausgeprägte, relativ scharfe Kanten ausgebildet werden.

Die freien C-Enden des Kapseläquatorringes 5 sind nach einwärts abgebogen und mit angeformten kleinen Ösen 6 versehen.

Die an die Ösen 6 anschließenden, jeweils etwa einen Viertelkreis bildenden Ringsegmente besitzen eine geringere radiale Dicke als ein etwa halbkreisförmiges mittleres Ringsegment, wobei sich die vorgenannten Ringsegmente in Richtung der Ösen 6 verjüngen können.

In Fig. 1 ist der im Kapselsack 3 implantierte Zustand des Kapseläquatorringes 5 mit durchgezogenen Linien dargestellt. In diesem Zustand sucht sich der Kapseläquatorring 5 federnd aufzuweiten. Mit strichlierten Linien ist in Fig. 1 der entspannte Zustand des Kapselspannringes 5 dargestellt.

Im übrigen sind in den Fig. 1 und 2 bevorzugte Bemessungen des Kapseläquatorringes angegeben, jeweils in mm, wobei Abweichungen von ± 0,05 mm ohne weiteres tolerierbar sind.

Grundsätzlich können jedoch auch abweichende Abmessungen vorgesehen sein.

Besonders vorteilhaft ist, daß die an der Innenseite des Kapselsackes 3 anliegende Außenumfangsfläche des Kapseläquatorringes 5 in Richtung der Ringachse eine vergleichsweise große Breite hat, welche bevorzugt bei etwa 5 bis 7 % des Durchmessers des Kapseläquatorringes 5 im implantierten Zustand liegt.

An den Ringkanten zwischen dem Außenumfang des Kapseläquatorringes 5 und dessen Stirnseiten wird der Kapselsack 3 gemäß Fig. 3 deutlich geknickt.

## Patentansprüche

1. Kapseläquatorring (5) in Form eines C-förmig offenen elastischen Federbügels, welcher in den eröffneten Kapselsack (3) eines Auges implantierbar ist und im implantierten Zustand mit seinem Außenumfang an der Innenseite des Kapselsackes an dessen Äquator anliegt und den Kapselsack radial stabilisiert, wobei die Enden des federbügelförmigen Kapseläquatorringes im implantierten Zustand gegen Federwiderstand aneinander angenähert sind, derart, daß sich der Kapseläquatorring im implantierten Zustand aufzuweiten sucht, mit relativ scharfkantig an die Stirnseiten des Ringes anschließendem, im wesentlichen zylindrischem Außenumfang und im wesentlichen rechteckigem Querschnitt,
**dadurch gekennzeichnet,**
**daß** die langen Rechteckseiten des Querschnittes parallel zur Ringachse erstreckt und die freien C-Enden des federbügelförmigen Kapseläquatorrings nach einwärts abgebogen sowie mit innenseitig des Ringes angeformten kleinen Ösen (6) versehen sind, und daß an die Ösen anschließende, jeweils etwa einen Viertelkreis bildende Ringsegmente eine geringere radiale Dicke als ein etwa halbkreisförmiges mittleres Ringsegment besitzt.

2. Kapseläquatorring nach Anspruch 1,
**dadurch gekennzeichnet,**
**daß** sich die Viertelkreis-Ringsegmente zu den Ösen (6) hin verjüngen.

3. Kapseläquatorring nach Anspruch 1 oder 2,
**dadurch gekennzeichnet,**
**daß** der Außenumfang quer zur Ring- bzw. Äquatorebene eine axiale Breite aufweist, welche größer als etwa 5 % % des Durchmessers des implantierten Kapseläquatorringes (5) bzw. des ausgespannten Äquators ist.

4. Kapseläquatorring nach einem der Ansprüche 1 bis 3,
**dadurch gekennzeichnet,**
**daß** die axiale Breite des Außenumfangs zwischen etwa 5 und 7 % des Durchmessers des Kapseläquatorringes (5) im implantierten Zustand liegt.

5. Kapseläquatorring nach einem der Ansprüche 1 bis 4,
**dadurch gekennzeichnet,**
**daß** die axiale Breite des Außenumfanges bzw. des Ringes bei etwa 0,7 mm liegt.

6. Kapseläquatorring nach einem der Ansprüche 1 bis 5,
**dadurch gekennzeichnet,**
**daß** die radiale Dicke des Kapseläquatorringes (5) in einem mittleren Bereich zwischen den freien Enden bei ca. 0,20±0,05 mm und an den den freien Enden benachbarten Bereichen bei etwa 0,15±0,05 mm liegt.

7. Kapseläquatorring nach einem der Ansprüche 1 bis 6,
bestehend aus PMMA (Polymethylmethacrylat) bzw. Acrylglas.

## Claims

1. Capsular equatorial ring (5) in the form of an elastic spring clip which is open in the shape of a C, which ring can be implanted into the opened capsular bag (3) of an eye and, in the implanted state, bears with its outer circumference against the inside of the capsular bag at the equator of the latter and radially stabilizes the capsular bag, the ends of the capsular equatorial ring in the form of a spring clip in the implanted state being brought close together counter to spring resistance in such a way that, in the implanted state, the capsular equatorial ring attempts to open out, having a substantially cylindrical outer circumference adjoining with a relatively sharp edge the end faces of the ring and a substantially rectangular cross-section, **characterized in that** the long sides of the rectangle of the cross-section extends [sic] parallel to the axis of the ring and the free C ends of the capsular equatorial ring in the form of a spring clip are bent away inwards and provided with small eyelets (6) formed onto the inner side of the ring, and **in that** ring segments adjoining the eyelets and respectively forming approximately a quarter circle has [sic] a smaller radial thickness than an approximately semicircular middle ring segment.

2. Capsular equatorial ring according to Claim 1, **characterized in that** the quarter-circle ring segments taper towards the eyelets (6).

3. Capsular equatorial ring according to Claim 1 or 2, **characterized in that** the outer circumference has transversely with respect to the plane of the ring or of the equator an axial width which is greater than about 5% of the diameter of the implanted capsular equatorial ring (5) or of the extended equator.

4. Capsular equatorial ring according to one of Claims 1 to 3, **characterized in that** the axial width of the outer circumference is between about 5 and 7% of the diameter of the capsular equatorial ring (5) in the implanted state.

5. Capsular equatorial ring according to one of Claims 1 to 4, **characterized in that** the axial width of the outer circumference or of the ring is about 0.7 mm.

6. Capsular equatorial ring according to one of Claims 1 to 5, **characterized in that** the radial thickness of the capsular equatorial ring (5) in a middle region between the free ends is about 0.20 ± 0.05 mm, and at the regions adjacent to the free ends is about 0.15 ± 0.05 mm.

7. Capsular equatorial ring according to one of Claims 1 to 6, consisting of PMMA (polymethyl methacrylate) or acrylic glass.

## Revendications

1. Anneau équatorial pour capsule cristallinienne (5) en forme d'étrier élastique ouvert en C, lequel peut être implanté dans la capsule cristallinienne (3) ouverte d'un oeil et, à l'état implanté, repose par son pourtour extérieur sur le côté intérieur de la capsule cristallinienne à l'équateur de celle-ci et stabilise radialement la capsule cristallinienne, dans lequel les extrémités de l'anneau équatorial pour capsule cristallinienne en forme d'étrier élastique, à l'état implanté, sont rapprochés l'une de l'autre contre la résistance élastique, de manière telle qu'à l'état implanté, l'anneau équatorial pour capsule cristallinienne cherche à s'ouvrir, comportant un pourtour extérieur essentiellement cylindrique se raccordant aux faces frontales de l'anneau et une section transversale essentiellement rectangulaire, **caractérisé en ce que** les côtés longs du rectangle s'étendant parallèlement à l'axe annulaire et les extrémités libres du C de l'anneau équatorial pour capsule cristallinienne sont cintrées vers l'intérieur et pourvues de petits oeillets (6) formés, et **en ce que** les segments d'anneau, formant respectivement sensiblement un quart de cercle, se raccordant aux oeillets, possèdent une épaisseur radiale inférieure à un segment d'anneau central sensiblement de forme semi-circulaire.

2. Anneau équatorial pour capsule cristallinienne selon la revendication 1, **caractérisé en ce que** les segments d'anneau en quarts de cercle se rétrécissent dans la direction des oeillets (6).

3. Anneau équatorial pour capsule cristallinienne selon la revendication 1 ou 2, **caractérisé en ce que** le pourtour extérieur possède transversalement au plan annulaire ou équatorial une largeur axiale, laquelle est supérieure de sensiblement 5 % au diamètre de l'anneau équatorial pour capsule cristallinienne (5) implanté ou de l'équateur tendu.

4. Anneau équatorial pour capsule cristallinienne selon l'une des revendications 1 à 3, **caractérisé en ce que** la largeur axiale du pourtour extérieur est comprise entre sensiblement 5 et 7 % du diamètre de l'anneau équatorial pour capsule cristallinienne (5) à l'état implanté.

5. Anneau équatorial pour capsule cristallinienne selon l'une des revendications 1 à 4, **caractérisé en ce que** la largeur axiale du pourtour extérieur ou de l'anneau s'élève à sensiblement 0,7 mm.

6. Anneau équatorial pour capsule cristallinienne selon l'une des revendications 1 à 5, **caractérisé en ce que** l'épaisseur radiale de l'anneau équatorial pour capsule cristallinienne (5) se situe à environ 0,20 ± 0,05 mm dans une zone centrale entre les extrémités libres et à environ 0,15 ± 0,05 mm dans les zones adjacentes aux extrémités libres.

7. Anneau équatorial pour capsule cristallinienne selon l'une des revendications 1 à 6 consistant en PMMA (Polymethylmethacrylate) et/ou verre à acrylate (plexiglas).
